# EUROPEAN PATENT SPECIFICATION

(11) **EP 0 613 949 B1**
(45) Date of publication and mention of the grant of the patent: **29.12.2004**
(21) Application number: 94250046.3
(22) Date of filing: 23.02.1994
(51) Int. Cl.: C12N 15/60, C12N 9/88, C12P 21/08, A61K 38/51, G01N 33/68

(54) **Gene encoding chondroitinase ABC and uses therefor**
Gen für Chondroitinase ABC und dessen Verwendungen
Gène codant pour la chondroitinase ABC et usages de celui-ci

(30) Priority: 24.02.1993 JP 3581093
(43) Date of publication of application: 07.09.1994
(73) Proprietor: MARUHA CORPORATION, Chiyoda-ku Tokyo (JP)
(72) Inventor: Sato, Nobuyuki, Tsuchiura, Ibaraki (JP); Shimada, Masahiko, Kashiwa, Chiba (JP); Oda, Hiroshi, Toyonaka, Osaka (JP)
(74) Representative: Ziebig, Marlene, Dr. Dipl.-Chem.

(56) References cited:
- EP-A- 0 355 831
- EP-A- 0 576 294
- WO-A-91/16070
- US-A- 4 696 816
- J. BIOL. CHEM. vol. 243 , 1968 pages 1523 - 1535 YAMAGATA, T. ET AL. 'Purification and Properties of Bacterial Chondroitinases and Chondrosulfatases'
- CLIN. ORTHOP. vol. 253 , 1990 pages 301 - 308 FUMIHIKO KATO ET AL. 'Experimental Chemonucleolysis with Chondroitinase ABC'
- J. BIOL. CHEM. vol. 243 , 1968 pages 1536 - 1542 HIDEIKO SAITO ET AL. 'Enzymatic Methods for the Determination of Small Quantities of Isomeric Chondroitine Sulfates'
- ANAL. BIOCHEM. vol. 113, no. 2 , 1981 pages 416 - 422 BREEN, M. ET AL. 'Microanalysis of Glycosaminoglycans'
- APPL. MICROBIOL. BIOTECHNOL. vol. 41, no. 1 , 1994 pages 39 - 46 SATO, N. ET AL. 'Cloning and expression in E. coli of the gene encoding Proteus vulgaris chondroitin ABC lyase'
- J. OF NEUROSCIENCE vol. 11, no. 11 , 1991 pages 3398 - 3411 MCKEON, R., J. ET AL. 'Reduction of Neurite Outgrowth...'

### SEQUENCE LISTING

(1) GENERAL INFORMATION:
   (i) APPLICANT: SATO, Nobuyuki, SHIMADA, Masahiko; and ODA, Hiroshi
   (ii) TITLE OF INVENTION: GENE ENCODING CHONDROITINASE ABC AND USES THEREFOR
   (iii) NUMBER OF SEQUENCES: 17
   (iv) CORRESPONDENCE ADDRESS:
      (A) ADDRESSEE: LAHIVE & COCKFIELD
      (B) STREET: 60 STATE STREET, SUITE 510
      (C) CITY: BOSTON
      (D) STATE: MASSACHUSETTS
      (E) COUNTRY: USA
      (F) ZIP: 02109
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: ASCII text
   (vi) CURRENT APPLICATION DATA:
      (A) APPLICATION NUMBER:
      (B) FILING DATE: 8-JUN-1993
      (C) CLASSIFICATION:
   (vii) PRIOR APPLICATION DATA:
      (A) APPLICATION NUMBER: JP 5-35810
      (B) FILING DATE: 24-FEB-1993
   (viii) ATTORNEY/AGENT INFORMATION:
      (A) NAME: Mandragouras, Amy E.
      (B) REGISTRATION NUMBER: 36,207
      (C) REFERENCE/DOCKET NUMBER: ITI-003
   (ix) TELECOMMUNICATION INFORMATION:
      (A) TELEPHONE: (617) 227-7400
      (B) TELEFAX: (617) 227-5941
(2) INFORMATION FOR SEQ ID NO:1:
   (ii) SEQUENCE CHARACTERISTICS:
      (A) LENGTH: 3486 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 225..3287
   (xi) SEQUENCE DESCRIPTION: SEQ ID NO:1:
   ATCAATCAAC GCCACAGCCT TACCTATTTT AATACAGGGG GAAGTACCTT TGATATTAAA     60
   GGAAATACCG TTGGTGGTGA CATTATTAGT GCGGAATTAG GTGCAAATCT CGATATCACT    120
   CAATCATTAA ATTTAGGCAC AACGATGGGC TATCAGCGTT ATGACAAATT TAATGAAGGA    180
   CGCATTGGTT TCACTGTTAG CCAGCGTTTC TAAGGAGAAA AATA ATG CCG ATA TTT      236
                                                    Met Pro Ile Phe
                                                     1
   CGT TTT ACT GCA CTT GCA ATG ACA TTG GGG CTA TTA TCA GCG CCT TAT      284
   Arg Phe Thr Ala Leu Ala Met Thr Leu Gly Leu Leu Ser Ala Pro Tyr
    5                  10                  15                  20
   AAC GCG ATG GCA GCC ACC AGC AAT CCT GCA TTT GAT CCT AAA AAT CTG      332
   Asn Ala Met Ala Ala Thr Ser Asn Pro Ala Phe Asp Pro Lys Asn Leu
                   25                  30                  35
   ATG CAG TCA GAA ATT TAC CAT TTT GCA CAA AAT AAC CCA TTA GCA GAC      380
   Met Gln Ser Glu Ile Tyr His Phe Ala Gln Asn Asn Pro Leu Ala Asp
               40                  45                  50
   TTC TCA TCA GAT AAA AAC TCA ATA CTA ACG TTA TCT GAT AAA CGT AGC      428
   Phe Ser Ser Asp Lys Asn Ser Ile Leu Thr Leu Ser Asp Lys Arg Ser
           55                  60                  65
   ATT ATG GGA AAC CAA TCT CTT TTA TGG AAA TGG AAA GGT GGT AGT AGC      476
   Ile Met Gly Asn Gln Ser Leu Leu Trp Lys Trp Lys Gly Gly Ser Ser
       70                  75                  80
   TTT ACT TTA CAT AAA AAA CTG ATT GTC CCC ACC GAT AAA GAA GCA TCT      524
   Phe Thr Leu His Lys Lys Leu Ile Val Pro Thr Asp Lys Glu Ala Ser
    85                  90                  95                 100
   AAA GCA TGG GGA CGC TCA TCT ACC CCC GTT TTC TCA TTT TGG CTT TAC   572
   Lys Ala Trp Gly Arg Ser Ser Thr Pro Val Phe Ser Phe Trp Leu Tyr
                   105                 110                 115
   AAT GAA AAA CCG ATT GAT GGT TAT CCT ACT ATC GAT TTC GGA GAA AAA   620
   Asn Glu Lys Pro Ile Asp Gly Tyr Pro Thr Ile Asp Phe Gly Glu Lys
               120                 125                 130
   CTC ATT TCA ACC AGT GAG GCT CAG GCA GGC TTT AAA GTA AAA TTA GAT   668
   Leu Ile Ser Thr Ser Glu Ala Gln Ala Gly Phe Lys Val Lys Leu Asp
           135                 140                 145
   TTC ACT GGC TGG CGT GCT GTG GGA GTC TCT TTA AAT AAC GAT CTT GAA   716
   Phe Thr Gly Trp Arg Ala Val Gly Val Ser Leu Asn Asn Asp Leu Glu
       150                 155                 160
   AAT CGA GAG ATG ACC TTA AAT GCA ACC AAT ACC TCC TCT GAT GGT ACT   764
   Asn Arg Glu Met Thr Leu Asn Ala Thr Asn Thr Ser Ser Asp Gly Thr
   165                 170                 175                 180
   CAA GAC AGC ATT GGG CGT TCT TTA GGT GCT AAA GTC GAT AGT ATT CGT   812
   Gln Asp Ser Ile Gly Arg Ser Leu Gly Ala Lys Val Asp Ser Ile Arg
                   185                 190                 195
   TTT AAA GCG CCT TCT AAT GTG AGT CAG GGT GAA ATC TAT ATC GAC CGT   860
   Phe Lys Ala Pro Ser Asn Val Ser Gln Gly Glu Ile Tyr Ile Asp Arg
               200                 205                 210
   ATT ATG TTT TCT GTC GAT GAT GCT CGC TAC CAA TGG TCT GAT TAT CAA   908
   Ile Met Phe Ser Val Asp Asp Ala Arg Tyr Gln Trp Ser Asp Tyr Gln
           215                 220                 225
   GTA AAA ACT CGC TTA TCA GAA CCT GAA ATT CAA TTT CAC AAC GTA AAG   956
   Val Lys Thr Arg Leu Ser Glu Pro Glu Ile Gln Phe His Asn Val Lys
       230                 235                 240
   CCA CAA CTA CCT GTA ACA CCT GAA AAT TTA GCG GCC ATT GAT CTT ATT  1004
   Pro Gln Leu Pro Val Thr Pro Glu Asn Leu Ala Ala Ile Asp Leu Ile
   245                 250                 255                 260
   CGC CAA CGT CTA ATT AAT GAA TTT GTC GGA GGT GAA AAA GAG ACA AAC  1052
   Arg Gln Arg Leu Ile Asn Glu Phe Val Gly Gly Glu Lys Glu Thr Asn
                   265                 270                 275
   CTC GCA TTA GAA GAG AAT ATC AGC AAA TTA AAA AGT GAT TTC GAT GCT  1100
   Leu Ala Leu Glu Glu Asn Ile Ser Lys Leu Lys Ser Asp Phe Asp Ala
               280                 285                 290
   CTT AAT ATT CAC ACT TTA GCA AAT GGT GGA ACG CAA GGC AGA CAT CTG  1148
   Leu Asn Ile His Thr Leu Ala Asn Gly Gly Thr Gln Gly Arg His Leu
           295                 300                 305
   ATC ACT GAT AAA CAA ATC ATT ATT TAT CAA CCA GAG AAT CTT AAC TCC 1196
   Ile Thr Asp Lys Gln Ile Ile Ile Tyr Gln Pro Glu Asn Leu Asn Ser
   310 315 320
   CAA GAT AAA CAA CTA TTT GAT AAT TAT GTT ATT TTA GGT AAT TAC ACG 1244
   Gln Asp Lys Gln Leu Phe Asp Asn Tyr Val Ile Leu Gly Asn Tyr Thr
   325 330 335 340
   ACA TTA ATG TTT AAT ATT AGC CGT GCT TAT GTG CTG GAA AAA GAT CCC 1292
   Thr Leu Met Phe Asn Ile Ser Arg Ala Tyr Val Leu Glu Lys Asp Pro
   345 350 355
   ACA CAA AAG GCG CAA CTA AAG CAG ATG TAC TTA TTA GTG ACA AAG CAT 1340
   Thr Gln Lys Ala Gln Leu Lys Gln Met Tyr Leu Leu Val Thr Lys His
   360 365 370
   TTA TTA GAT CAA GGC TTT GTT AAA GGG AGT GCT TTA GTG ACA ACC CAT 1388
   Leu Leu Asp Gln Gly Phe Val Lys Gly Ser Ala Leu Val Thr Thr His
   375 380 385
   CAC TGG GGA TAC AGT TCT CGT TGG TGG TAT ATT TCC ACG TTA TTA ATG 1436
   His Trp Gly Tyr Ser Ser Arg Trp Trp Tyr Ile Ser Thr Leu Leu Met
   390 395 400
   TCT GAT GCA CTA AAA GAA GCG AAC CTA CAA ACT CAA GTT TAT GAT TCA 1484
   Ser Asp Ala Leu Lys Glu Ala Asn Leu Gln Thr Gln Val Tyr Asp Ser
   405 410 415 420
   TTA CTG TGG TAT TCA CGT GAG TTT AAA AGT AGT TTT GAT ATG AAA GTA 1532
   Leu Leu Trp Tyr Ser Arg Glu Phe Lys Ser Ser Phe Asp Met Lys Val
   425 430 435
   AGT GCT GAT AGC TCT GAT CTA GAT TAT TTC AAT ACC TTA TCT CGC CAA 1580
   Ser Ala Asp Ser Ser Asp Leu Asp Tyr Phe Asn Thr Leu Ser Arg Gln
   440 445 450
   CAT TTA GCC TTA TTA TTA CTA GAG CCT GAT GAT CAA AAG CGT ATC AAC 1628
   His Leu Ala Leu Leu Leu Leu Glu Pro Asp Asp Gln Lys Arg Ile Asn
   455 460 465
   TTA GTT AAT ACT TTC AGC CAT TAT ATC ACT GGC GCA TTA ACG CAA GTG 1676
   Leu Val Asn Thr Phe Ser His Tyr Ile Thr Gly Ala Leu Thr Gln Val
   470 475 480
   CCA CCG GGT GGT AAA GAT GGT TTA CGC CTG ATG GTA CAG CAT GGC GAC 1724
   Pro Pro Gly Gly Lys Asp Gly Leu Arg Leu Met Val Gln His Gly Asp
   485 490 495 500
   ATG AAG GCA ACT ATC CGG GTT ACT CTT TCC CAG CCT TTA AAA ATG CCT 1772
   Met Lys Ala Thr Ile Arg Val Thr Leu Ser Gln Pro Leu Lys Met Pro
   505 510 515
   CTC AGC TTA TTT ATT TAT TAC GCG ATA CAC CAT TTC CAG TTG GGT GAA 1820
   Leu Ser Leu Phe Ile Tyr Tyr Ala Ile His His Phe Gln Leu Gly Glu
   520 525 530
   AGT GGT TGG AAT AAC CTG AAA AAA GCG ATG GTT TCA GCG TGG ATC TAC 1868
   Ser Gly Trp Asn Asn Leu Lys Lys Ala Met Val Ser Ala Trp Ile Tyr
   535 540 545
   AGT AAT CCA GAA GTT GGA TTA CCG CTT GCA GGA AGA CAC CCT TTT AAC 1916
   Ser Asn Pro Glu Val Gly Leu Pro Leu Ala Gly Arg His Pro Phe Asn
   550 555 560
   TCA CCT TCG TTA AAA TCA GTC GCT CAA GGC TAT TAC TGG CTT GCC ATG 1964
   Ser Pro Ser Leu Lys Ser Val Ala Gln Gly Tyr Tyr Trp Leu Ala Met
   565 570 575 580
   TCT GCA AAA TCA TCG CCT GAT AAA ACA CTT GCA TCT ATT TAT CTT GCG 2012
   Ser Ala Lys Ser Ser Pro Asp Lys Thr Leu Ala Ser Ile Tyr Leu Ala
   585 590 595
   ATT AGT GAT AAA ACA CAA AAT GAA TCA ACT GCT ATT TTT GGA GAA ACT 2060
   Ile Ser Asp Lys Thr Gln Asn Glu Ser Thr Ala Ile Phe Gly Glu Thr
   600 605 610
   ATT ACA CCA GCG TCT TTA CCT CAA GGT TTC TAT GCC TTT AAT GGC GGT 2108
   Ile Thr Pro Ala Ser Leu Pro Gln Gly Phe Tyr Ala Phe Asn Gly Gly
   615 620 625
   GCT TTT GGT ATT CAT CGT TGG CAA GAT AAA ATG GTG ACA CTG AAA GCT 2156
   Ala Phe Gly Ile His Arg Trp Gln Asp Lys Met Val Thr Leu Lys Ala
   630 635 640
   TAT AAC ACC AAT GTT TGG TCA TCT GAA ATT TAT AAC AAA GAT AAC CGT 2204
   Tyr Asn Thr Asn Val Trp Ser Ser Glu Ile Tyr Asn Lys Asp Asn Arg
   645 650 655 660
   TAT GGC CGT TAC CAA AGT CAT GGT GTC GGT CAA ATA GTG AGT AAT GGC 2252
   Tyr Gly Arg Tyr Gln Ser His Gly Val Gly Gln Ile Val Ser Asn Gly
   665 670 675
   TCG CAG CTT TCA CAG GGC TAT CAG CAA GAA GGT TGG GAT TGG AAT AGA 2300
   Ser Gln Leu Ser Gln Gly Tyr Gln Gln Glu Gly Trp Asp Trp Asn Arg
   680 685 690
   ATG CAA GGG GCA ACC ACT ATT CAC CTT CCT CTT AAA GAC TTA GAC AGT 2348
   Met Gln Gly Ala Thr Thr Ile His Leu Pro Leu Lys Asp Leu Asp Ser
   695 700 705
   CCT AAA CCT CAT ACC TTA ATG CAA CGT GGA GAG CGT GGA TTT AGC GGA   2396
   Pro Lys Pro His Thr Leu Met Gln Arg Gly Glu Arg Gly Phe Ser Gly
       710                 715                 720
   ACA TCA TCC CTT GAA GGT CAA TAT GGC ATG ATG GCA TTC GAT CTT ATT   2444
   Thr Ser Ser Leu Glu Gly Gln Tyr Gly Met Met Ala Phe Asp Leu Ile
   725                 730                 735                 740
   TAT CCC GCC AAT CTT GAG CGT TTT GAT CCT AAT TTC ACT GCG AAA AAG   2492
   Tyr Pro Ala Asn Leu Glu Arg Phe Asp Pro Asn Phe Thr Ala Lys Lys
                   745                 750                 755
   AGT GTA TTA GCC GCT GAT AAT CAC TTA ATT TTT ATT GGT AGC AAT ATA   2540
   Ser Val Leu Ala Ala Asp Asn His Leu Ile Phe Ile Gly Ser Asn Ile
               760                 765                 770
   AAT AGT AGT GAT AAA AAT AAA AAT GTT GAA ACG ACC TTA TTC CAA CAT   2588
   Asn Ser Ser Asp Lys Asn Lys Asn Val Glu Thr Thr Leu Phe Gln His
           775                 780                 785
   GCC ATT ACT CCA ACA TTA AAT ACC CTT TGG ATT AAT GGA CAA AAG ATA   2636
   Ala Ile Thr Pro Thr Leu Asn Thr Leu Trp Ile Asn Gly Gln Lys Ile
       790                 795                 800
   GAA AAC ATG CCT TAT CAA ACA ACA CTT CAA CAA GGT GAT TGG TTA ATT   2684
   Glu Asn Met Pro Tyr Gln Thr Thr Leu Gln Gln Gly Asp Trp Leu Ile
   805                 810                 815                 820
   GAT AGC AAT GGC AAT GGT TAC TTA ATT ACT CAA GCA GAA AAA GTA AAT   2732
   Asp Ser Asn Gly Asn Gly Tyr Leu Ile Thr Gln Ala Glu Lys Val Asn
                   825                 830                 835
   GTA AGT CGC CAA CAT CAG GTT TCA GCG GAA AAT AAA AAT CGC CAA CCG   2780
   Val Ser Arg Gln His Gln Val Ser Ala Glu Asn Lys Asn Arg Gln Pro
               840                 845                 850
   ACA GAA GGA AAC TTT AGC TCG GCA TGG ATC GAT CAC AGG ACT CGC CCC   2828
   Thr Glu Gly Asn Phe Ser Ser Ala Trp Ile Asp His Arg Thr Arg Pro
           855                 860                 865
   AAA GAT GCC AGT TAT GAG TAT ATG GTC TTT TTA GAT GCG ACA CCT GAA   2876
   Lys Asp Ala Ser Tyr Glu Tyr Met Val Phe Leu Asp Ala Thr Pro Glu
       870                 875                 880
   AAA ATG GGA GAG ATG GCA CAA AAA TTC CGT GAA AAT AAT GGG TTA TAT   2924
   Lys Met Gly Glu Met Ala Gln Lys Phe Arg Glu Asn Asn Gly Leu Tyr
   885                 890                 895                 900
   CAG GTT CTT CGT AAG GAT AAA GAC GTT CAT ATT ATT CTC GAT AAA CTC   2972
   Gln Val Leu Arg Lys Asp Lys Asp Val His Ile Ile Leu Asp Lys Leu
                   905                 910                 915
   AGC AAT GTA ACG GGA TAT GCC TTT TAT CAG CCA GCA TCA ATT GAA GAC     3020
   Ser Asn Val Thr Gly Tyr Ala Phe Tyr Gln Pro Ala Ser Ile Glu Asp
               920                 925                 930
   AAA TGG ATC AAA AAG GTT AAT AAA CCT GCA ATT GTG ATG ACT CAT CGA     3068
   Lys Trp Ile Lys Lys Val Asn Lys Pro Ala Ile Val Met Thr His Arg
           935                 940                 945
   CAA AAA GAC ACT CTT ATT GTC AGT GCA GTT ACA CCT GAT TTA AAT ATG     3116
   Gln Lys Asp Thr Leu Ile Val Ser Ala Val Thr Pro Asp Leu Asn Met
       950                 955                 960
   ACT CGC CAA AAA GCA GCA ACT CCT GTC ACC ATC AAT GTC ACG ATT AAT     3164
   Thr Arg Gln Lys Ala Ala Thr Pro Val Thr Ile Asn Val Thr Ile Asn
   965                 970                 975                 980
   GGC AAA TGG CAA TCT GCT GAT AAA AAT AGT GAA GTG AAA TAT CAG GTT     3212
   Gly Lys Trp Gln Ser Ala Asp Lys Asn Ser Glu Val Lys Tyr Gln Val
                   985                 990                 995
   TCT GGT GAT AAC ACT GAA CTG ACG TTT ACG AGT TAC TTT GGT ATT CCA     3260
   Ser Gly Asp Asn Thr Glu Leu Thr Phe Thr Ser Tyr Phe Gly Ile Pro
              1000                1005                1010
   CAA GAA ATC AAA CTC TCG CCA CTC CCT TGATTTAATC AAAAGGAACC           3307
   Gln Glu Ile Lys Leu Ser Pro Leu Pro
              1015                1020
   TCTTGCGTTC CTTTTTTATT TGCAGGAAAT CTGATTATGC TAATAAAAAA              3357
   CCCTTTAGCC CAGGCGGTTA CATTAAGCCT CTGTTTATCA TTACCCGCAC              3407
   AAGCATTACC CACTCTGTCT CATGAAGCTT TCGGCGTATT TATCTTTTGA              3457
   AGGTGAATTA CCCAATACCT TACCACTTC                                     3486
(3) INFORMATION FOR SEQ ID NO:2:
   (i) SEQUENCE CHARACTERISTICS:
      (A) LENGTH: 1021 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Pro Ile Phe Arg Phe Thr Ala Leu Ala Met Thr Leu Gly Leu Leu
    1               5                  10                  15
   Ser Ala Pro Tyr Asn Ala Met Ala Ala Thr Ser Asn Pro Ala Phe Asp
               20                  25                  30
   Pro Lys Asn Leu Met Gln Ser Glu Ile Tyr His Phe Ala Gln Asn Asn
           35                  40                  45
   Pro Leu Ala Asp Phe Ser Ser Asp Lys Asn Ser Ile Leu Thr Leu Ser
       50                  55                  60
   Asp Lys Arg Ser Ile Met Gly Asn Gln Ser Leu Leu Trp Lys Trp Lys
   65                  70                  75                  80
   Gly Gly Ser Ser Phe Thr Leu His Lys Lys Leu Ile Val Pro Thr Asp
                   85                  90                  95
   Lys Glu Ala Ser Lys Ala Trp Gly Arg Ser Ser Thr Pro Val Phe Ser
               100                 105                 110
   Phe Trp Leu Tyr Asn Glu Lys Pro Ile Asp Gly Tyr Pro Thr Ile Asp
           115                 120                 125
   Phe Gly Glu Lys Leu Ile Ser Thr Ser Glu Ala Gln Ala Gly Phe Lys
       130                 135                 140
   Val Lys Leu Asp Phe Thr Gly Trp Arg Ala Val Gly Val Ser Leu Asn
   145                 150                 155                 160
   Asn Asp Leu Glu Asn Arg Glu Met Thr Leu Asn Ala Thr Asn Thr Ser
                   165                 170                 175
   Ser Asp Gly Thr Gln Asp Ser Ile Gly Arg Ser Leu Gly Ala Lys Val
               180                 185                 190
   Asp Ser Ile Arg Phe Lys Ala Pro Ser Asn Val Ser Gln Gly Glu Ile
           195                 200                 205
   Tyr Ile Asp Arg Ile Met Phe Ser Val Asp Asp Ala Arg Tyr Gln Trp
       210                 215                 220
   Ser Asp Tyr Gln Val Lys Thr Arg Leu Ser Glu Pro Glu Ile Gln Phe
   225                 230                 235                 240
   His Asn Val Lys Pro Gln Leu Pro Val Thr Pro Glu Asn Leu Ala Ala
                   245                 250                 255
   Ile Asp Leu Ile Arg Gln Arg Leu Ile Asn Glu Phe Val Gly Gly Glu
               260                 265                 270
   Lys Glu Thr Asn Leu Ala Leu Glu Glu Asn Ile Ser Lys Leu Lys Ser
           275                 280                 285
   Asp Phe Asp Ala Leu Asn Ile His Thr Leu Ala Asn Gly Gly Thr Gln
   290 295 300
   Gly Arg His Leu Ile Thr Asp Lys Gln Ile Ile Ile Tyr Gln Pro Glu
   305 310 315 320
   Asn Leu Asn Ser Gln Asp Lys Gln Leu Phe Asp Asn Tyr Val Ile Leu
   325 330 335
   Gly Asn Tyr Thr Thr Leu Met Phe Asn Ile Ser Arg Ala Tyr Val Leu
   340 345 350
   Glu Lys Asp Pro Thr Gln Lys Ala Gln Leu Lys Gln Met Tyr Leu Leu
   355 360 365
   Val Thr Lys His Leu Leu Asp Gln Gly Phe Val Lys Gly Ser Ala Leu
   370 375 380
   Val Thr Thr His His Trp Gly Tyr Ser Ser Arg Trp Trp Tyr Ile Ser
   385 390 395 400
   Thr Leu Leu Met Ser Asp Ala Leu Lys Glu Ala Asn Leu Gln Thr Gln
   405 410 415
   Val Tyr Asp Ser Leu Leu Trp Tyr Ser Arg Glu Phe Lys Ser Ser Phe
   420 425 430
   Asp Met Lys Val Ser Ala Asp Ser Ser Asp Leu Asp Tyr Phe Asn Thr
   435 440 445
   Leu Ser Arg Gln His Leu Ala Leu Leu Leu Leu Glu Pro Asp Asp Gln
   450 455 460
   Lys Arg Ile Asn Leu Val Asn Thr Phe Ser His Tyr Ile Thr Gly Ala
   465 470 475 480
   Leu Thr Gln Val Pro Pro Gly Gly Lys Asp Gly Leu Arg Leu Met Val
   485 490 495
   Gln His Gly Asp Met Lys Ala Thr Ile Arg Val Thr Leu Ser Gln Pro
   500 505 510
   Leu Lys Met Pro Leu Ser Leu Phe Ile Tyr Tyr Ala Ile His His Phe
   515 520 525
   Gln Leu Gly Glu Ser Gly Trp Asn Asn Leu Lys Lys Ala Met Val Ser
   530 535 540
   Ala Trp Ile Tyr Ser Asn Pro Glu Val Gly Leu Pro Leu Ala Gly Arg
   545 550 555 560
   His Pro Phe Asn Ser Pro Ser Leu Lys Ser Val Ala Gln Gly Tyr Tyr
   565 570 575
   Trp Leu Ala Met Ser Ala Lys Ser Ser Pro Asp Lys Thr Leu Ala Ser
   580 585 590
   Ile Tyr Leu Ala Ile Ser Asp Lys Thr Gln Asn Glu Ser Thr Ala Ile
   595 600 605
   Phe Gly Glu Thr Ile Thr Pro Ala Ser Leu Pro Gln Gly Phe Tyr Ala
   610 615 620
   Phe Asn Gly Gly Ala Phe Gly Ile His Arg Trp Gln Asp Lys Met Val
   625 630 635 640
   Thr Leu Lys Ala Tyr Asn Thr Asn Val Trp Ser Ser Glu Ile Tyr Asn
   645 650 655
   Lys Asp Asn Arg Tyr Gly Arg Tyr Gln Ser His Gly Val Gly Gln Ile
   660 665 670
   Val Ser Asn Gly Ser Gln Leu Ser Gln Gly Tyr Gln Gln Glu Gly Trp
   675 680 685
   Asp Trp Asn Arg Met Gln Gly Ala Thr Thr Ile His Leu Pro Leu Lys
   690 695 700
   Asp Leu Asp Ser Pro Lys Pro His Thr Leu Met Gln Arg Gly Glu Arg
   705 710 715 720
   Gly Phe Ser Gly Thr Ser Ser Leu Glu Gly Gln Tyr Gly Met Met Ala
   725 730 735
   Phe Asp Leu Ile Tyr Pro Ala Asn Leu Glu Arg Phe Asp Pro Asn Phe
   740 745 750
   Thr Ala Lys Lys Ser Val Leu Ala Ala Asp Asn His Leu Ile Phe Ile
   755 760 765
   Gly Ser Asn Ile Asn Ser Ser Asp Lys Asn Lys Asn Val Glu Thr Thr
   770 775 780
   Leu Phe Gln His Ala Ile Thr Pro Thr Leu Asn Thr Leu Trp Ile Asn
   785 790 795 800
   Gly Gln Lys Ile Glu Asn Met Pro Tyr Gln Thr Thr Leu Gln Gln Gly
   805 810 815
   Asp Trp Leu Ile Asp Ser Asn Gly Asn Gly Tyr Leu Ile Thr Gln Ala
   820 825 830
   Glu Lys Val Asn Val Ser Arg Gln His Gln Val Ser Ala Glu Asn Lys
   835 840 845
   Asn Arg Gln Pro Thr Glu Gly Asn Phe Ser Ser Ala Trp Ile Asp His
   850 855 860
   Arg Thr Arg Pro Lys Asp Ala Ser Tyr Glu Tyr Met Val Phe Leu Asp
   865 870 875 880
   Ala Thr Pro Glu Lys Met Gly Glu Met Ala Gln Lys Phe Arg Glu Asn
   885 890 895
   Asn Gly Leu Tyr Gln Val Leu Arg Lys Asp Lys Asp Val His Ile Ile
   900 905 910
   Leu Asp Lys Leu Ser Asn Val Thr Gly Tyr Ala Phe Tyr Gln Pro Ala
   915 920 925
   Ser Ile Glu Asp Lys Trp Ile Lys Lys Val Asn Lys Pro Ala Ile Val
   930 935 940
   Met Thr His Arg Gln Lys Asp Thr Leu Ile Val Ser Ala Val Thr Pro
   945 950 955 960
   Asp Leu Asn Met Thr Arg Gln Lys Ala Ala Thr Pro Val Thr Ile Asn
   965 970 975
   Val Thr Ile Asn Gly Lys Trp Gln Ser Ala Asp Lys Asn Ser Glu Val
   980 985 990
   Lys Tyr Gln Val Ser Gly Asp Asn Thr Glu Leu Thr Phe Thr Ser Tyr
   995 1000 1005
   Phe Gly Ile Pro Gln Glu Ile Lys Leu Ser Pro Leu Pro
   1010 1015 1020
(4) INFORMATION FOR SEQ ID NO:3:
   (i) SEQUENCE CHARACTERISTICS:
      (A) LENGTH: 18 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:3:
   Ala Thr Ser Asn Pro Ala Phe Asp Pro Lys Asn Leu Met Gln Ser Glu
   1 5 10 15
   Ile Tyr
(5) INFORMATION FOR SEQ ID NO:4 :
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:4:
   CTAATGGGTT ATTTTGTGCA A 21
(6) INFORMATION FOR SEQ ID NO: 5
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:5:
   GCNACNUCNA AYCCNGC 17
(7) INFORMATION FOR SEQ ID NO: 6:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:6:
   GCNACNAGYA AYCCNGC 17
(8) INFORMATION FOR SEQ ID NO:7:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:7:
   UACGUYAGNC UYUADAU 17
(9) INFORMATION FOR SEQ ID NO:8:
   (i) SEQUENCE CHARACTERISTICS:
      (A) LENGTH: 17 base pairs
      (B) TYPE: nucleic acid
      (C) STRANDEDNESS: single
      (D) TOPOLOGY : linear
   (ii) MOLECULE TYPE: cDNA
   (xi) SEQUENCE DESCRIPTION: SEQ ID NO:8:
   UACGUYUCRC UYUADAU 17
(10) INFORMATION FOR SEQ ID NO:9:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:9:
   CATTTGATCC TAAAAATCTG ATGCA 25
(11) INFORMATION FOR SEQ ID NO:10:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: CDNA
   (xi) SEQUENCE DESCRIPTION: SEQ ID NO:10:
   GCAACCAGCA ATCCTGCA 18
(12) INFORMATION FOR SEQ ID NO:11:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:11:
   GACTACGTCA GGCTTTTAAT 20
(2) INFORMATION FOR SEQ ID NO:12:
   (i) SEQUENCE CHARACTERISTICS:
      (A) LENGTH: 53 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:12:
   GCAACCAGCA ATCCTGCATT TGATCCTAAA AATCTGATGC AGTCCGAAAT TTA 53
(2) INFORMATION FOR SEQ ID NO:13:
   (i) SEQUENCE CHARACTERISTICS:
      (A) LENGTH: 53 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:13:
   TAATTTTCGG ACTGCATCAG ATTTTTAGGA TCAAATGCAG GATTGCTGGT TGC 53
(2) INFORMATION FOR SEQ ID NO:14:
   (i) SEQUENCE CHARACTERISTICS:
      (A) LENGTH: 122 base pairs
      (B) TYPE: nucleic acid
      (C) STRANDEDNESS: single
      (D) TOPOLOGY: circular
   (ii) MOLECULE TYPE: other
   (xi) SEQUENCE DESCRIPTION: SEQ ID NO:14:
   GATATCACTC AATCATTAAA TTTAGGCACA ACGATGGGCT ATCAGCGTTA TGACAAATTT   60
   AATGAAGGAC GCATTGGTTT CACTGTTAGC CAGCGTTTCT AAGGAGAAAA ATAATGCCGA  120
   TA                                                                 122
(2) INFORMATION FOR SEQ ID NO:15:
   (i) SEQUENCE CHARACTERISTICS:
      (A) LENGTH: 60 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:15:
   ATGACAAATT TAATGAAGGA CGCATTGGTT TCACTGTTAG CCAGCGTTTC TAAGGAGAAA   60
(2) INFORMATION FOR SEQ ID NO:16:
   (i) SEQUENCE CHARACTERISTICS:
      (A) LENGTH: 400 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 296..400
   (xi) SEQUENCE DESCRIPTION: SEQ ID NO:16:
   CAGACTGCTT ATGGCAAATT AACCCCCTCT CTTAATCTTC GTTATTCAAA GATATTGCAG   60
   GTGACAATGA TATCAATCAA CGCCACAGCC TTACCTATTT TAATACAGGG GGAAGTACCT  120
   TTGATATTAA AGGAAATACC GTTGGTGGTG ACATTATTAG TGCGGAATTA GGTGCAAATC  180
   TCGATATCAC TCAATCATTA AATTTAGGCA CAACGATGGG CTATCAGCGT TATGACAAAT  240
   TTAATGAAGG ACGCATTGGT TTCACTGTTA GCCAGCGTTT CTAAGGAGAA AAATA ATG   298
                                                                Met
                                                                  1
   CCG ATA TTT CGT TTT ACT GCA CTT GCA ATG ACA TTG GGG CTA TTA TCA    346
   Pro Ile Phe Arg Phe Thr Ala Leu Ala Met Thr Leu Gly Leu Leu Ser
                   5                  10                  15
   GCG CCT TAT AAC GCG ATG GCA GCC ACC AGC AAT CCT GCA TTT GAT GCT    394
   Ala Pro Tyr Asn Ala Met Ala Ala Thr Ser Asn Pro Ala Phe Asp Ala
           20                  25                  30
   AAA AAT                                                            400
   Lys Asn
       35
(2) INFORMATION FOR SEQ ID NO: 17:
   (i) SEQUENCE CHARACTERISTICS:
      (A) LENGTH: 35 amino acids
      (B) TYPE: amino acid
      (D) TOPOLOGY: linear
   (ii) MOLECULE TYPE: protein
   (xi) SEQUENCE DESCRIPTION: SEQ ID NO:17:
   Met Pro Ile Phe Arg Phe Thr Ala Leu Ala Met Thr Leu Gly Leu Leu
    1               5                  10                  15
   Ser Ala Pro Tyr Asn Ala Met Ala Ala Thr Ser Asn Pro Ala Phe Asp
               20                  25                  30
   Ala Lys Asn
           35

## Description

### Background of the Invention

Chondroitin lyase (EC 4.2.2.4) or chondroitinase ABC is an enzyme which catalyzes the depolymerization of chondroitin sulfate. Through β-elimination of 1,4 hexosaminidic bonds, chondroitinase ABC degrades chondroitin, chondroitin 4-sulfate (chondroitin A sulfate), dermatan sulfate (chondroitin B sulfate), chondroitin 6-sulfate (chondroitin C sulfate) and hyaluronate to the respective unsaturated disaccharides (Δdi-OS for chondroitin, Δdi-4S for chondroitin A sulfate, Δdi-4-6S for chondroitin B sulfate and Δdi-6S for chondroitin C sulfate, respectively). The enzyme has been isolated in various strains of bacteria (Neuberg, C. et al., (1914) Biochem. Z. 67: 82-89) (Neuberg, C. et al. (1931) Biochem. Z. 234: 345-346; Yamagata, T. et al., (1968) J. Biol. Chem. 243: 1523-1535) including *Proteus vulgaris* (Yamagata, T. et al. (1968) J. Biol. Chem. 243: 1523-1535; Thurston, C.F. (1974) J. Gen. Microbiol. 80:515-522; Sato N. et al, (1986) Agric. Biol. Chem, 50: 1057-1059; Sato N. et al, (1986) Biotechnol. Bioeng. 28: 1707-1712; Sato, N. et al. (1986) J. Ferment. Technol, 64: 155-159).

Chondroitin sulfate consists of alternating β 1-3 glucuronidic and β 1-4 N-acetylgalactosaminidic bonds, and is sulfated at either C-4 or C-6 of the N-acetylgalactosamine pyranose. Chondroitin sulfate is known to be widely distributed in mammalian tissue, such as in skin, cornea, bone and especially in cartilage. Thus, chondroitinase ABC has been used as an experimental reagent for the determination or quantitation of total amount of galactosaminoglycans in the field of orthopedic surgery (Linker, A. et al. (1960) J. Biol. Chem.235: 3061-3065; Saito, H. et al, (1968) J. Biol. Chem. 243: 1536-1542; Pettipher, E. R. et al, (1989) Arthritis Rheum. 32: 601-607; Caterson, B. et al. (1990) J. Cell Science 97: 411-417; and Seibel, M. J. et al, (1992) Arch. Biochem. Biophys. 296: 410-418).

Recently, chondroitinase ABC has been reported to be a potential reagent for chemonucleolysis, an established treatment for intervertebral disc displacement (Kato, F. et al. (1990) Clin. Orthop. 253: 301-308; Henderson, N. et al, (1991) Spine 16: 203-209). However, for the utilization of chondroitinase ABC as a clinical reagent, there are many problems to be overcome. For example, the preparation of chondroitinase ABC from *P. vulgaris* require tedious and intricate procedures, since the cellular content of the enzyme is low.

EP 0355 831 A2 describes a method of purification of glycoseaminoglycan degrading enzymes, for example of chondroitinase ABC from *P. vulgaris* (NCTC 4636). The method comprises chromatographically treating under use of an insoluble sulphated polysaccharide carrier. The specific activity of the isolated chondroitinase ABC was increased as compared before chromatography.

### Summary of the Invention

This invention pertains an isolated nucleic acid sequence of SEQ IQ NO. 1 coding for a gene with chondroitinase ABC activity and an isolated protein with chondroitinase ABCactivity consisting of SEQ ID NO: 2 produced in a host cell transformed with a nucleic acid vector directing the expression of the nucleotide sequence coding for the protein of SEQ ID NO:2. The protein with chondroitinase ABC activity consisting of SEQ ID No. 2 prepared by chemical synthesis is also provided. The isolated protein of SEQ ID NO: 2 can be used in methods of treating intervertebral disc displacement and promoting neurite regeneration or in method of detecting the presence of galactosaminoglycans.

### Brief Description of the Drawings

Figure 1-A and 1-B show the primers used for polymerase chain reaction (PCR) amplification of chondroitinase ABC from *P. vulgaris* genomic DNA; Figure 1-B also shows the probe used for plaque hybridization; and Figure 1-C shows the restriction maps for three recombinant phages and the fragment of phage 11-5 which was subcloned into pSTV29 for sequencing.
Figure 2 shows the construction of pCHSP, a hybrid plasmid containing the putative promoter region of chondroitinase ABC (SEQ ID NO:14).
Figure 3 shows primer extension analysis using a sequencing ladder (SEQ ID NO:15).
Figure 4 shows the nucleotide sequence of the promoter region of chondroitinase ABC (SEQ ID NO: 16) and the peptide sequence. (SEQ ID NO:17).
Figure 5 shows the construction of plasmids pCHS 6, pCHSΔ 6, and pCHS 26 each of which contains a fragment of the chondroitinase ABC gene.
Figure 6 shows SDS-PAGE and immunoblot analysis of recombinant chondroitinase ABC protein produced by pCHSΔ 6 transformed *E. coli* (lane 1); protein produced by pSTV 29 without the chondroitinase ABC gene in *E. coli* (lane 2); natural chondroitinase ABC produced by *P. vulgaris* (lane 3); and molecular weight markers (lane 4).
Figure 7 shows the DNA (SEQ ID NO:1) and amino acid sequence (SEQ ID NO:2) of the chondroitinase ABC similar gene including non-coding regions.

### Detailed Description of the Invention

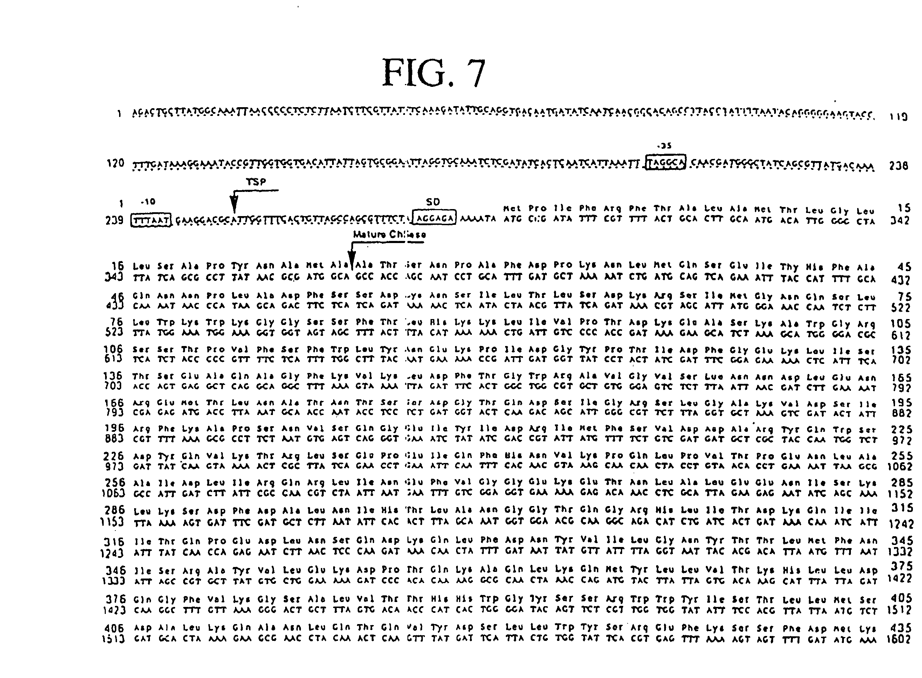

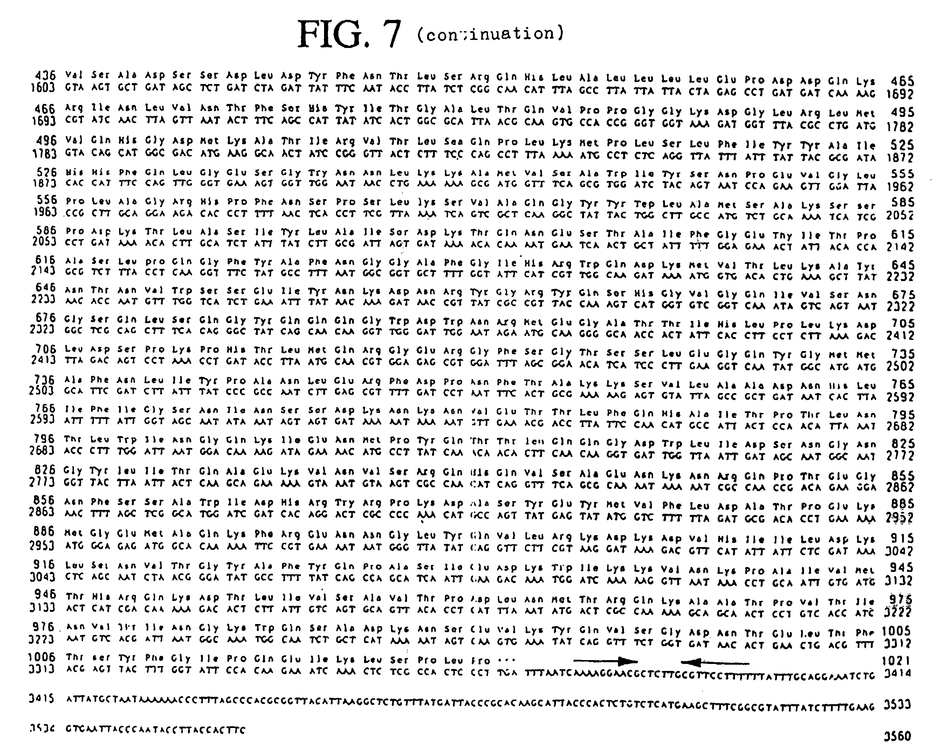

This invention pertains to a nucleic acid sequence of SEQ ID NO:1 coding for a protein with chondroitinase ABC activity of SEQ ID NO: 2 an enzyme which degrades chondroitin A, B, and C. The gene was derived using recombinant DNA techniques. The nucleic acid sequence has the sequence shown in SEQ ID NO:1 (Fig.7). The deduced amino acid sequences of the protein is shown in SEQ ID NO:2 (Fig. 7)

The nucleic acid sequence coding for the protein can obtained from mRNA present in *Proteus vulgaris,* or can also be obtained from *P. vulgaris* genomic DNA. The nucleic acid sequence coding for the protein can obtained using the method disclosed herein or any other suitable technique for isolation and molecular cloning of genes. The nucleic acid is a DNA having the sequence depicted in SEQ ID NO:1 (Fig. 7).

This invention also provides expression vectors containing the nucleic acid sequence coding for the protein of SEQ ID NO:2, operably linked to at least one regulatory sequence. Operably linked is intended to mean that the nucleotides sequence is linked to a regulatory sequence in a manner which allows expression of the nucleotide sequence. Regulatory sequences are art-recognized and are selected to direct expression of chondroitinase ABC. Accordingly, the term regulatory sequence includes promoters, enhancers and other expression control elements. Such regulatory sequences are described in Goeddel, Gene Expression Technology: Methods in Enzymology 185, Academic Press, San Diego, CA (1990). It should be understood that the design of the expression vector may depend on such factors as the choice of the host cell to be transformed and/or the type of protein desired to be expressed.

This invention further pertains to a host cell transformed to express the protein of SEQ ID NO:2. The host cell may be any prokaryotic or eukaryotic cell. For example, the protein may be expressed in bacterial cells such as *E. coli*, insect cells (baculovirus), yeast, or mammalian cells such as Chinese hamster ovary cells (CHO). Other suitable host cells may be found in Goeddel, (1990) supra or one known to those skilled in the art.

Expression in eukaryotic cells such as mammalian, yeast, or insect cells can lead to partial or complete glycosylation and/or formation of relevant *inter*- or intra-chain disulfide bonds of recombinant protein
Examples of vectors for expression in yeast S. cerivisae include pYepSec 1 (Baldari et al., (1987) EMBO J, 6:229-234), pMFa (Kurjan and Herskowitz, (1982) Cell 30:933-943), pJRY88 (Schutz et al., (1987) Gene 54:113-123), and pYES2 (Invitrogen Corporation, San Diego, CA). Baculovirus vectors available for expression of proteins in cultured insect cells (SF 9 cells) include the pAc series (Smith et al., (1983) Mol. Cell Biol. 3:2156-2165) and the pVL series (Lucklow, V.A., and Summers, M.D., (1989) Virology 170:31-39). Generally COS cells (Gluzman, Y., (1981) Cell 23:175-182) are used in conjunction with such vectors as pCDM 8 (Aruffo, A. and Seed, B., (1987) Proc. Natl. Acad. Sci. USA 84:8573-8577) for transient amplification/expression in mammalian cells, while CHO (dhfr⁻ Chinese Hamster Ovary) cells are used with vectors such as pMT2PC (Kaufmann et al. (1987), EMBO J, 6:187-195) for stable amplification/expression in mammalian cells. Vector DNA can be introduced into mammalian cells via conventional techniques such as calcium phosphate or calcium chloride co-precipitation, DEAE-dextran-mediated transfection, or electroporation. Suitable methods for transforming host cells can be found in Sambrook et al., Molecular Cloning: A Laboratory Manual, 2nd Edition, Cold Spring Harbor Laboratory press (1989), and other laboratory textbooks.

Expression in prokaryotes is most often carried out in *E. coli* with either fusion or non-fusion inducible expression vectors. Fusion vectors usually add a number of NH₂ terminal amino acids to the expressed target gene. These NH₂ terminal amino acids often are referred to as a reporter group. Such reporter groups usually serve two purposes: 1) to increase the solubility of the target recombinant protein; and 2) to aid in the purification of the target recombinant protein by acting as a ligand in affinity purification. Often, in fusion expression vectors, a proteolytic cleavage site is introduced at the junction of the reporter group and the target recombinant protein to enable separation of the target recombinant protein from the reporter group subsequent to purification of the fusion protein. Such enzymes include Factor Xa, thrombin and enterokinase. Typical fusion expression vectors include pGEX (Amrad Corp., Melbourne, Australia), pMAL (New England Biolabs, Beverly, MA) and pRIT5 (Pharmacia, Piscataway, NJ) which fuse glutathione S-tranferase, maltose E binding protein, or protein A, respectively, to the target recombinant protein.

Inducible non-fusion expression vectors include pTrc (Amann et al., (1988) Gene 69:301-315) and pET 11d (Studier et al., Gene Expression Technology: Methods in Enzymology 185, Academic Press, San Diego, California (1990) 60-89). While target gene expression relies on host RNA polymerase transcription from the hybrid trp-lac fusion promoter in pTrc, expression of target genes inserted into pET 11d relies on transcription from the T7 gn10-lac 0 fusion promoter mediated by coexpressed viral RNA polymerase (T7 gn1). This viral polymerase is supplied by host strains BL2I (DE3) or HMS 174(DE3) from a resident g prophage harboring a T7 gnl under the transcriptional control of the lacUV 5 promoter

One strategy to maximize recombinant chondroitinase ABC expression in *E. coli* is to express the protein in a host bacteria with an impaired capacity to proteolytically cleave the recombinant protein (Gottesman, S., Gene Expression Technology: Methods in Enzymology 185, Academic Press, San Diego, California (1990) 119-128). Another strategy would be to alter the nucleic acid sequence of the chondroitinase ABC gene to be inserted into an expression vector so that the individual codons for each amino acid would be those preferentially utilized in highly expressed *E. coli* proteins (Wada et al. (1992) Nuc. Acids Res. 20:2111-2118). Such alteration of nucleic acid sequences of the invention can be carried out by standard DNA synthesis techniques.

The nucleic acid sequence (SEQ ID NO:1) of the invention can also be chemically synthesized using standard techniques. Various methods of chemically synthesizing polydeoxynucleotides are known, including solid-phase synthesis which, like peptide synthesis, has been fully automated in commercially available DNA synthesizers (See e.g., Itakura et al., U.S. Patent No. 4,598,049; Caruthers et al., U.S. Patent No. 4,458,066; and Itakura U.S. Patent Nos. 4,401,796 and 4,373,071, incorporated by reference herein).

This invention further pertains to methods of producing the protein of SEQ ID NO:2. For example, a host cell transformed with a nucleic acid vector directing expression of the nucleotide sequence coding for the protein can be cultured under appropriate conditions to allow expression of this protein with chondroitinase ABC activity to occur. The protein may be secreted and isolated from a mixture of cells and medium containing the protein. Alternatively, the protein may be retained cytoplasmically and the cells harvested, lysed and the protein isolated. The culture includes host cells, media and other byproducts. Suitable mediums for cell culture are well known in the art. The protein of SEQ ID NO:2 can be isolated from cell culture medium, host cells, or both using techniques known in the art for purifying proteins including ion-exchange chromatography, gel filtration chromatography, ultrafiltration, electrophoresis, and immunoaffinity purification with antibodies specific for said protein or fragment thereof.

Another aspect of the invention pertains to isolated protein of SEQ ID NO:2.

To facilitate purification and potentially increase solubility of the protein, it is possible to add an amino acid reporter group to the protein backbone. For example, hexa-histidine can be added to the protein for purification by immobilized metal ion affinity chromatography (Hochuli, E. et al., (1988) Bio/Technology 6:1321-1325). In addition, to facilitate isolation of the protein free of irrelevant sequences, specific endoprotease cleavage sites can be introduced between the sequences of the reporter group and the protein or peptide.

Antibodies which are not subject-matter of this invention can be used to isolated the naturally-occurring or native form of the protein or to neutralize the enzyme so that it is unable to depolymerize chondroitin.

For example, by using the isolated protein of SEQ ID NO:2 based on the cDNA sequence, anti-protein/anti-peptide antisera or monoclonal antibodies can be made using standard methods. A mammal such as a mouse, a hamster or a rabbit can be immunized with an immunogenic form of the isolated protein (e.g., the protein or an antigenic fragment which is capable of eliciting an antibody response). Techniques for conferring immunogenicity on a protein or peptide include conjugation to carriers or other techniques well known in the art. The protein can be administered in the presence of adjuvant. The progress of immunization can be monitored by detection of antibody titers in plasma or serum standard ELISA or other immunoassay can be used with the immunogen as antigen to assess the levels of antibodies.

Following immunization, antisera can be obtained and, if desired, polyclonal antibodies isolated from the serum. To produce monoclonal antibodies, antibody producing cells (lymphocytes) can be harvested from an immunized animal and fused by standard somatic cell fusion procedures with immortalizing cells such as myeloma cells to yield hybridoma cells. Such techniques are well known in the art, for example the hybridoma technique originally developed by Kohler and Milstein, Nature (1975) 256:495-497, as well as other techniques such as the human B-cell hybridoma technique (Kozbar et al., Immunology Today (1983) 4:72) and the EBV-hybridoma technique to produce human monoclonal antibodies (Cole et al., Monoclonal Antibodies and Cancer Therapy (1985) Alan R. Liss, Inc. pp. 77-96). Hybridoma cells can be screened immunochemically for production of antibodies specifically reactive with the protein of SEQÏD NO:2 and the monoclonal antibodies isolated.

The term antibody as used herein is intended to include fragments thereof which are also specifically reactive with the protein or fragment thereof. Antibodies can be fragmented using conventional techniques and the fragments screened for utility in the same manner as described above for whole antibodies. For example, F(ab')₂ fragments can be generated by treating the antibody with pepsin. The resulting F(ab')₂ fragment can be treated with papain to reduce disulfide bridges to produce Fab'fragments. Such an antibody is further intended to include bispecific and chimeric molecules having an anti-protein portion.

The use of the protein of SEQ ID NO:2 may be happened in therapeutic compositions for the treatment of intervertebral displacement or nerve damage. Such a composition can comprise a therapeutically active amount of the protein of SEQ ID NO:2 and a pharmaceutically acceptable carrier. Administration of the therapeutic compositions to an individual to be treated can be carried out using known procedures, at dosages and for periods of time effective to depolymerize chondroitin A, B or C. A therapeutically active amount of the protein may vary according to factors such as the amount of chondroitin to be eliminated, the age, sex, and weight of the individual, and the ability of the protein to depolymerize the chondroitin. Dosage regima may be adjusted to provide the optimum therapeutic response.

The active compound may be administered in a convenient manner such as by injection (subcutaneous, intravenous, etc.). If the active compound is administered by injection, for example, about 100 units of active compound per dosage unit may be administered to treat intervertebral disc displacement. One unit is the amount of enzyme needed to mediate the release of one micromole of 4,5 unsaturated disaccharide from a substrate of chondroitin C sulfate per minute at 37°C, pH 6.0.

The active compound may be administered parenterally. Dispersions can be prepared in glycerol, liquid polyethylene glycols, and mixtures thereof and in oils. Under ordinary conditions of storage and use, these preparations may contain a preservative to prevent the growth of microorganisms.

Pharmaceutical compositions suitable for injectable use include sterile aqueous solutions (where water soluble) or dispersions and sterile powders for the extemporaneous preparation of sterile injectable solutions or dispersion. In all cases, the composition must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminium monostearate or gelatin.

Sterile injectable solutions can be prepared by incorporating active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient (protein) plus any additional desired ingredient from a previously sterile-filtered solution thereof.

As used herein "pharmaceutically acceptable carrier" includes any and all solvents, dispersion media, antibacterial and antifungal agents, isotonic and absorption delaying agents, and the like. The use of such media and agents for pharmaceutically active substances is well known in the art. Except insofar as any conventional media or agent is incompatible with the active compound, use thereof in the therapeutic composition is contemplated. Supplementary active compounds can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the elimination of chondroitin A, B, or C.

Isolated protein of SEQ ID NO:2 (produced recombinantly or by chemical synthesis) is essentially free of all other *P. vulgaris* proteins. Such protein is of a consistent, well-defined composition and biological activity for use in preparations which can be administered for therapeutic purposes (e.g., to treat intervertebral disc displacement). The protein can also be used as diagnostic reagents or in the study of the mechanism of chondroitinase ABC and to design modified derivatives or analogs useful in the depolymerization of chondroitin.

In a method of treating intervertebral disc displacement by chemonucleolysis the isolated protein can be used, too. It is a particularly useful enzyme for the selective chemonucleolysis of the nucleus pulposus (See, for example, U.S. Patent No. 4,696,816). The nucleus pulposus is made up of proteoglycans and collagen fibers. The protein attacks the polysaccharide side chains of the proteoglycans and reduces the swelling of the disc without affecting the structural collagen components or degrading the protein element of the proteoglycan. The disc then shrinks and pressure on the spinal cord is relieved. Thus, to treat intervertebral disc displacement, an active amount of the protein of the invention can be applied to the affected area. For example, 100 units of isolated protein can be injected into the center of a disc by the standard technique of intradiscal injection (Brown, Intradiscal Therapy, Year Book Meidal Publishers, Inc., Chicago, 1983).

In a method of treating nerve damage an active amount of the protein of the invention can be used to the affected area to degrade chondroitin 6-sulfate proteoglycans. It has been found that chondroitin 6-sulfate proteoglycans inhibit regeneration of neurites in the adult vertebrate central nervous system (McKeon et al., J. Neurosci 11:3398-3411 (1991)). By removing chondroitin 6-sulfate proteoglycans from the point of injury, it is possible to promote neurite regeneration. For example, a therapeutically effective amount of the protein can be applied to the point of injury in an individual to degrade inhibitory chondroitin 6-sulfate proteoglycans. More than one dose may be administered as indicated by the exigencies of the therapeutic situation.

The protein of SEQ ID NO:2 of the invention can also be used as a diagnostic reagent for detecting the presence of a galactosaminoglycan, such as chondroitin sulfate. For example, this protein can be used as a reagent for determining or quantitating the amount of galactosaminoglycan in a mammalian tissue, such as skin, cornea, bone or cartilage (See e.g., Linker, A. et al. (1960) J. Biol.Chem. 235: 3061-3065; Saito, H. et al. (1968) J. Biol. Chem. 243: 1536-1542; Pettipher, E.R. at al:, (1989) Arthritis Rheum. 32: 601-607; Caterson, B. et. al. (1990) J. Cell Science 97: 411-417; and Seibel, M. J. et al. (1992) Arch. Biochem. Biophys. 296: 410-418). To determine the presence of chondroitin sulfate in a mammalian tissue, the protein can be contacted with a sample of the tissue and the presence or amount of chondroitin sulfate determined using methods well known in the art.

The invention is further illustrated by the following examples which should not be construed as further limiting the subject invention. The following methods and materials were used throughout the examples discussed below.

### Materials and Methods

Bacterial strains, plasmid and phage *P. vulgaris* IFO3988 was provided by the Institute for Fermentation, Osaka, Japan. *E. coli* P2392 (hsdR514(r^{k-}, m^{k+}), supE44, supF58, lacY1 or (lacIZY), galT22, metB1, trpR55, (P2)) was used as the lysogen for P2 phage. EMBL3 vector was purchased from Toyobo Co., Ltd., Japan. PCR products were ligated with pT7 Blue T-vector(Takara Shuzo Co., Ltd., Japan). *E. coli* JM109(recA1, endA1, gyrA96, thi, hsdR17(r^{k-}, m^{k+}), supE44, relA1, λ-, Δ(lac-proB), (F', proAB, lacIq M15, traD36) was used as the host strain for pMC1871 promoter selection vector (Pharmacia LKB, Japan). *E. coli* XL1-Blue(endA1, hsdR17(r^{k-}, m^{k+}), supE44, thi-1, recA1, gyrA96, relA1, Δ(lac), (F', proAB, lacI, (lacZΔM15, Tn10(tet^{r})) (Int'l Dep. No. FERM BP-4170). *E. coli* XL1-Blue is a host cell for both pSTV28, and pSTV29 (Takara Shuzo Co., Ltd., Japan).

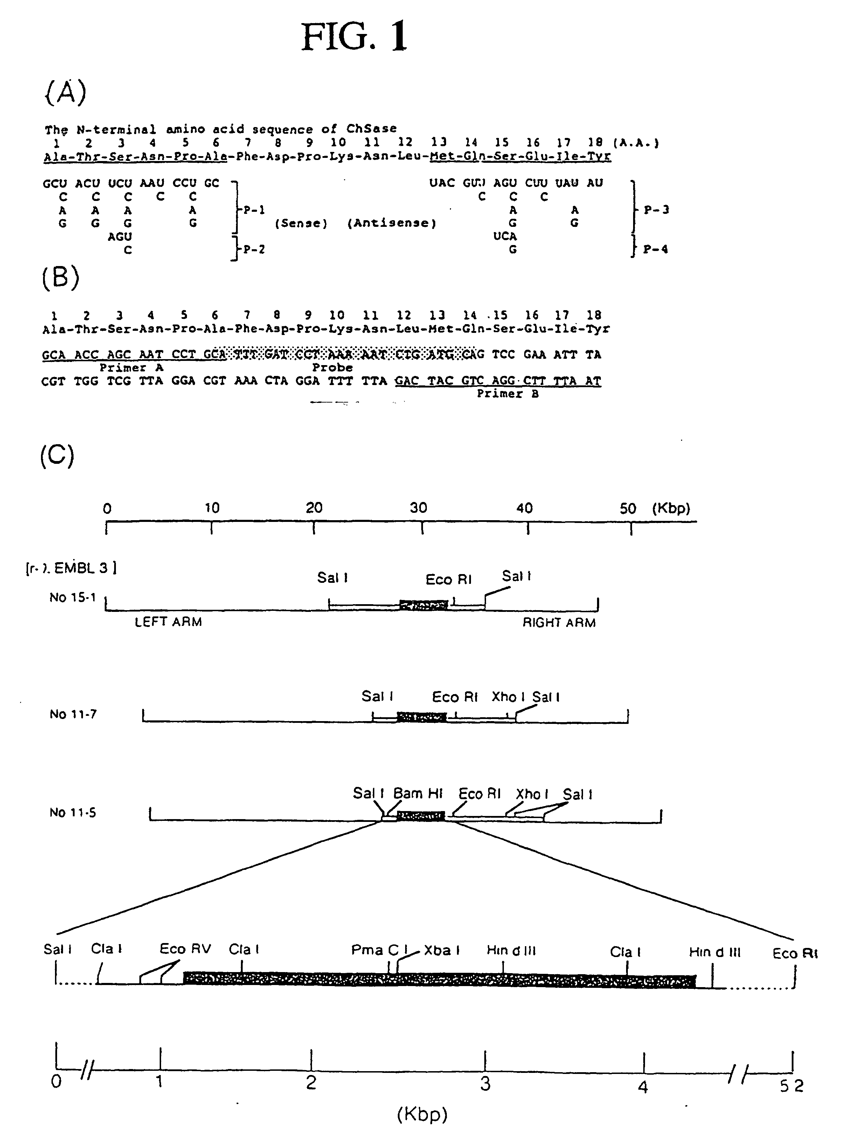

N-terminal amino acid sequence Chondroitinase ABC was purified as described previously (Sato, N. et al. (1986) Agric. Biol. Chem. 50: 1057-1059). The N-terminal amino acid sequence of chondroitinase ABC was sequenced by automatic Edman degradation on a gas-phase sequencer (Applied Biosystem, Foster, CA). The sequence of the N-terminal region of chondroitinase ABC was Ala-Thr-Ser-Asn-Pro-Ala-Phe-Asp-Pro-Lys-Asn-Leu-Met-Gln-Ser-Glu-Ile-Tyr (18 amino acid residues) (SEQ ID NO:3) The double stranded DNA sequence is shown in Figure 1-B (SEQ ID NOS:12-13).

Isolation of DNA and synthesis of nucleic acid, primer, and probe Isolation of chromosomal DNA of *P. vulgaris* was carried out by the standard method (Silhavy, T. J. et al. (1984) Experiments with Gene Fusion, Cold Spring Harbor Laboratory Press). Oligonucleotides used as primers and probe were synthesized with the DNA synthesizer, Cyclone Plus (Milligene/Biosearch, Bedford, MA).

Construction and screening of the gene library SauIII AI-partially digested fragments of total DNA were ligated to the BamHI site in λEMBL3 arms according to Frischauf et al. J. Mol. Biol. 170: 827-842 (1983)). The ligation mixture was packaged in vitro and transfected to *E. coli* P2392 according to the instructions of the suppliers (Stratagene, La Jolla, CA).

PCR amplification Primers for the chondroitinase ABC gene were designed according to the amino acid sequence of the chondroitinase ABC N-terminal region (SEQ ID NO:3)(Fig. 1-A). The primers were as follows
5'-GCNACNUCNAAYCCNGC-3' (P-1, sense)(SEQ ID NO:5);
5'-GCNACNAGYAAYCCNGC-3' (P-2, sense)(SEQ ID NO:6);
5'-UACGUYAGNCUYUADAU-3' (P-3, antisense)(SEQ ID NO:7);
5'-UACGUYUCRCUYUADAU-3' (P-4 antisense)(SEQ ID NO:8) (Fig. 1-A). PCR was performed using a GeneAmp Kit (Takara Shuzo Co., Ltd., Japan) in a final volume of 100 µl which contained: 1 µg of genomic DNA solution, 10 µl of 10x PCR reaction buffer, 16 µl of 1.25 mM dNTP mixture, 0.6 nmol of mixed primers and 2.5 units of Taq DNA polymerase (Takara Shuzo Co., Ltd., Japan). The mixture was subjected to PCR amplification using the DNA thermal cycler (GeneAmp PCR System 9600, Perkin-Elmer/Cetus, Norwalk, CT) for 28 cycles. Each cycle was 1 minute at 93°C(denaturation), 1.5 minutes at 50°C(annealing) and 0.5 minute at 72°C(elongation). PCR products were analyzed by electrophoresis through a 5% agarose gel (Nusieve GTG agarose, FMC Bioproducts, Rockford, ME) and the 54 bp fragment encoding 17 amino acids of N-terminal region was cut out of the gel. Gel-purified PCR products were directly cloned into pT7 Blue PCR vector.

DNA sequencing and isolation of the chondroitinase ABC gene Double-stranded plasmids purified by polyethylene glycol were denatured with alkali and sequenced by dideoxynucleotide chain termination method (Sanger, R. et al. (1977) Proc. Natl. Acad. Sci. USA 74: 5463-5467) using the sequence system, Hitachi WS10A Personal Sequencer (Hitachi Electronics Co., Ltd., Japan) Direct sequencing was done according to the method of Gyllesten & Erlich (Gyllensten, U. (1989) in PCR Technology, Erlich, H.A., Ed., Stockton Press, New York, pp. 45-60). PCR screening was carried out by the method of Olson et al.(Science 245: 1434-1435 (1989)). Plaque hybridization (Sambrook, J. et al. (1989) Molecular Cloning: A Laboratory Manual, 2nd ed., Cold Spring Harbor Laboratory Press) and Southern hybridization (Southern, E. M. (1975) J. Mol. Biol. 8: 503-517) were performed as outlined in the instructions of the supplier(Amersham Japan).

Primer extension analysis A 21-mer oligonucleotide(5'-CTA ATG GGT TAT TTT GTG CAA-3') (SEQ ID NO:4) complementary to the 5'-end (nucleotides 355-375) of the chondroitinase ABC gene was used as a primer. It was labeled with γ-³²P ATP (Amersham Japan) using polynucleotide kinase (Toyobo Co., Ltd., Japan). Total RNA of *P. vulgaris* was prepared according to the method of Aiba (J. Biol. Chem. 260: 3063-3070 (1985)). The labeled primer and 5 µg of total RNA were coprecipitated with ethanol. After annealing at 25°C for 6 hours in a hybridization buffer (80% formamide, 40 mM PIPES(pH 6.4), 1 mM EDTA and 400 mM NaCl), 250 mM NaCl, 50 mM sodium acetate(pH 4.6), 4.5 mM ZnSO₄, 100 µg/ml heat-denatured salmon testes DNA and 15 unit/µl reverse transcriptase of Rous associated virus 2 (Takara Shuzo Co., Ltd., Japan) were added to the mixture. The primer extension reaction was carried out at 37°C for 60 minutes.

Culture conditions Cells of *E. coli* XL1-Blue carrying recombinant plasmid were grown in 3 ml of LB broth(1% tryptone, 0.5% yeast extract, 1% NaCl, 25 µg/ml of chloramphenicol (pH 7.5)) at 37°C for 16 hr with reciprocation (120 rpm, 5 cm stroke). The cells were harvested by centrifugation and washed twice with 0.85% saline solution. Cells were transferred to 100 ml of chondroitin 6-sulfate (Taiyo Fishery Co., Ltd., Japan) medium(0.7% K₂HPO₄, 0.3% KH₂PO₄, 0.01% MgSO₄•7H₂O, 0.1% (NH₄)₂SO₄, 0.1% yeast extract, 0.3% chondroitin 6-sulfate, 0.01% glucose, 25 µg/ml chloramphenicol (pH 7.5)) or glucose medium (composition is the same as that of chondroitin medium except that glucose (0.3%) was used as a carbon source) to make a final concentration of A₆₁₀=0.05. After incubation for 3 days at 37°C with reciprocation, the cells were removed by centrifugation and degradation products of chondroitin 6-sulfate in the culture fluid were determined. The cells harvested from chondroitin and glucose media were washed twice with 50 mM Tris-HCl buffer (pH 8.0) and sonicated at 90 kHz for 5 minutes at 0°C. The cell debris were removed by centrifugation at 20,000g for 30 minutes, and the supernatant was used for the assay of chondroitinase ABC.

Enzyme assay Chondroitinase ABC was assayed as described previously (Sato, N. et al. (1986) J. Ferment. Technol. 64: 155-159). The assay mixture (3 ml) containing 0.5% chondroitin 6-sulfate, 100 mM potassium phosphate buffer (pH 8.0) and cell extract, was incubated at 37°C for 10 minutes, and the amount of N-acetylgalactosamine end group formed was determined by the method of Reissig (J. Biol. Chem. 217: 959-966 (1955)). Activity was expressed as the quantity of enzyme that catalyzed the formation of 1 µmol of unsaturated disaccharide (Δdi-6S) from chondroitin 6-sulfate per minute at 37 °C.

Western blot analysis IgG specific to chondroitinase ABC was isolated from antisera raised in guinea pig using the technique described previously (Sato, N. et al. (1988) Biotechnol. Appl. Biochem. 10: 385-393). Proteins in crude cell extracts prepared from *E. coli* transformant were separated by sodium dodecyl sulfate-polyacrylamide gel electrophoresis (SDS-PAGE) as described previously (Sato, N. et al. (1986) Agric. Biol. Chem. 50: 1057-1059). Western blotting procedures were described previously (Sato, N. et al. (1989) Appl. Microbiol. Biotechnol. 30: 153-159).

### Example 1

### Isolation and sequence determination of the chondroitinase ABC gene

According to the amino acid sequence of the N-terminal region of purified chondroitinase ABC (Ala-Thr-Ser-Asn-Pro-Ala-Phe-Asp-Pro-Lys-Asn-Leu-Met-Gln-Ser-Glu-Ile-Tyr (Fig. 1-A)(SEQ ID NO:3)), a set of degenerate oligo mixed primers (5'-GCNACNUCNAAYCCNGC-3' (P-1, sense)(SEQ ID NO:5); 5'-GCNACNAGYAAYCCNGC-3' (P-2, sense)(SEQ ID NO:6); 5'-UACGUYAGNCUYUADAU-3' (P-3, antisense)(SEQ ID NO:7); 5'-UACGUYUCRCUYUADAU-3' (P-4 antisense)(SEQ ID NO:8))(Fig. 1-A) were synthesized as follows. To determine the appropriate primers for sequencing, PCR amplification of a combination of primers P-1(SEQ ID NO:5), P-2(SEQ ID NO:6)(sense) and P-3(SEQ ID NO:7), P-4(SEQ ID NO:8)(antisense) was performed. After agarose gel electrophoresis of these PCR products, a 54 bp fragment was extracted and directly inserted into pT7 Blue PCR vector, and the inserted fragment was sequenced. The nucleotide sequence of this fragment was found to be identical to the N-terminal amino acid sequence (Fig. 1-B) (SEQ ID NO:3). Then, using primer A (5'-GCAACCAGCAATCCTGCA-3')(SEQ ID NO:10), primer B (5'-GACTACGTCAGGCTTTTAAT-3')(SEQ ID NO:11)(Fig. 1-B) and 1 µg of *P. vulgaris* genomic DNA as a template, PCR analysis was performed and PCR products were analyzed by agarose gel electrophoresis. No non-specific PCR products were observed.

We then diluted λEMBL3 recombinant phage stock library. The diluted library was used for PCR screening. An unique 54 bp fragment was clearly detected until the dilution of 1/10³(2 x 10⁵ pfu) phage stock solution as a template. The diluted phage solution was divided by 1/10(2 x 10⁴ pfu) and was infected into *E. coli* P2392. They were then subjected to plaque hybridization using ³²P-labeled probe (5'-CATTTGATCCTAAAAATCTGATGCA-3')(SEQ ID NO:9)(Fig. 1-B). The recombinant phages were chosen at random and analyzed by restriction mapping and Southern blotting. All phages contained common 4.2 kb EcoRV-EcoRI, 1.1 kb ClaI, and 2.0 kb EcoRV-HindIII fragments which hybridized strongly with the probe(SEQ ID NO:9). The restriction maps of three types of SalI fragments are shown in Fig. 1-C. Southern hybridization patterns of restricted genomic DNA from *P. vulgaris* matched the restriction map of these fragments. This result suggests that the 4.2 kb EcoRV-EcoRI fragment originated in the *P. vulgaris* genome, and therefore, the chondroitinase ABC gene exists as a single copy. When purified chondroitinase ABC from *P. vulgaris* was analyzed by SDS-PAGE, two types of chondroitinase ABC protein, one 100 kd protein and one subunit-like protein at 80 kd and 20 kd, were observed. The amino acid composition of the 100 kd protein and the subunit-like protein (80 kd and 20 kd) were quite similar, and the N-terminal amino acid sequences of the 100 kd and 20 kd proteins were identical. The results indicate that the two forms of chondroitinase ABC were not derived from two separate chondroitinase ABC genes.

The 5.2 kb SalI-EcoRI fragment in the recombinant λEMBL3 (No.11-5) (Fig. 1-C) was subcloned into pSTV29 for sequencing and the resulting hybrid plasmid was designated pCHS6. The entire 3,063 bp nucleotide sequence of the coding region for the chondroitinase ABC gene as well as 224 and 200 nucleotides of the upstream and downstream regions, respectively and the deduced amino acid sequence of chondroitinase ABC are shown in Figure 7 (SEQ ID NO:1). The 25-mer oligonucleotide probe (SEQ ID NO:9) hybridized to nucleotide 314-337. The 16/18 nucleotide of primer A and the 17/18 nucleotide of primer B were the same in nucleotides 297-313 and 333-349. The G+C content of the chondroitinase ABC gene was 38.6%. The open reading frame encoded a polypeptide with a molecular weight of 115,218, which represents a precursor polypeptide containing a signal peptide sequence that is subsequently cleaved off at Ala²⁴-Ala²⁵ during secretion of the mature chondroitinase ABC protein having a molecular weight of 112,365.

### Example 2

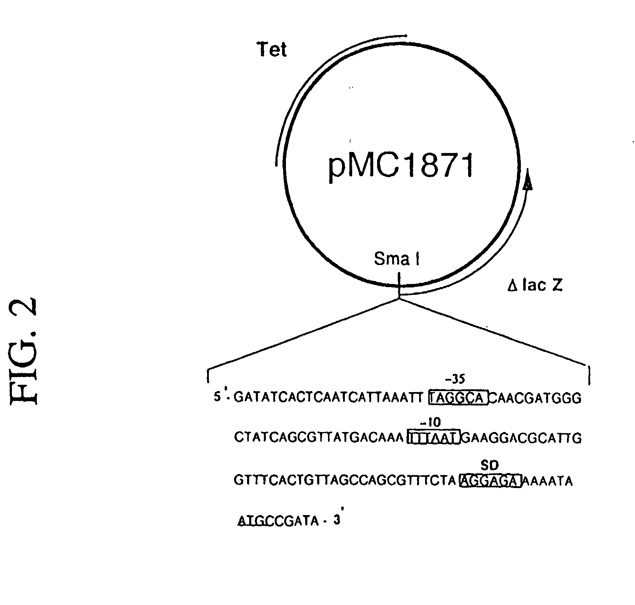

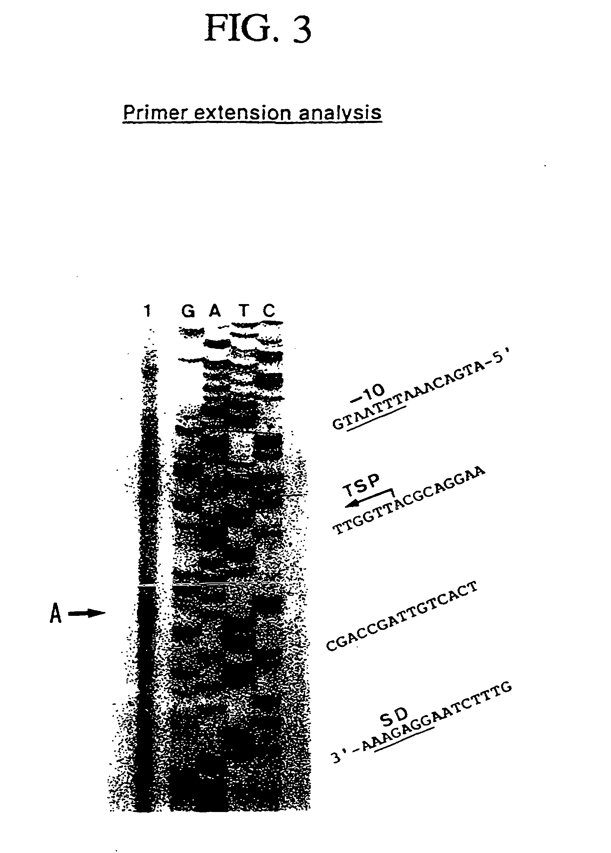

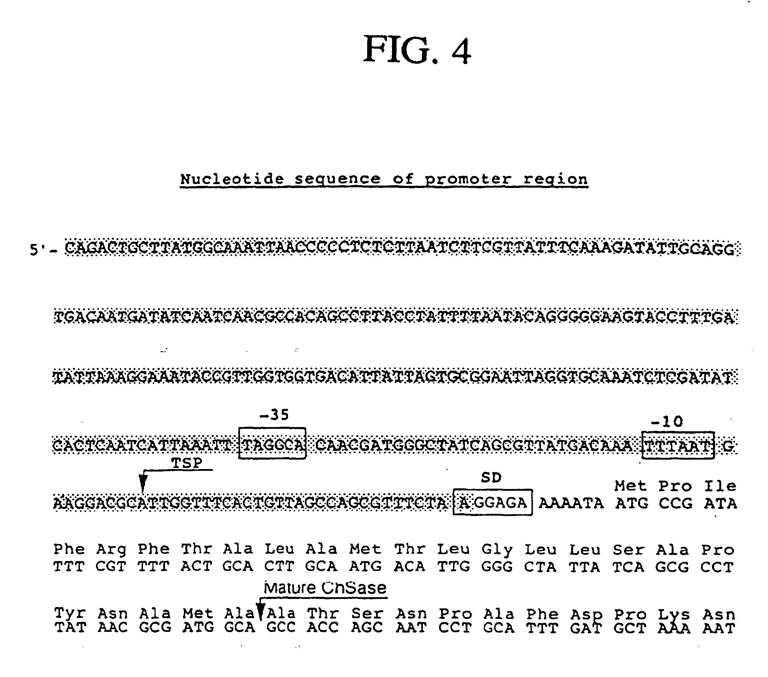

Analysis of the transcription region of the chondroitinase ABC gene In order to confirm the potential promoter region of the chondroitinase ABC gene, we amplified the region of nucleotide 112-283 using PCR. The PCR product was blunt-ended with T4 DNA polymerase and inserted into the SmaI site of the promoter selection vector, pMC1871, and the hybrid plasmid, designated pCHSP, was introduced into *E. coli* JM109 (Fig. 2)(SEQ ID NO:14). The transformant was then cultured in an LB medium containing 25 µg/ml tetracycline at 37°C for 14 hr, and β-galactosidase activity was assayed (Table I). Although the β-galactosidase activity of the *E. coli* transformant carrying pMC1871 was not detectable, the *E. coli* transformant carrying pCHSP produced β-galactosidase. This result indicates that the chondroitinase ABC gene can function as a promoter in *E. coli* cells. However, there is a possibility that the promoter recognized in *E. coli* cells may not be the promoter in *P. vulgaris.* To confirm that the promoter is recognized in *P. vulgaris*, primer extension analysis was carried out (Fig. 3) (SEQ ID NO:15). The transcription start point was localized to an adenosine 41 bp upstream from the start codon, ATG (Fig. 4) (SEQ ID NO:16). The potential pribnow box (TTTAAT) (nucleotides 169-174) was located 12 bp upstream from the transcription start point (Fig. 4) (SEQ ID NO:16). However, the -35 consensus sequence was not found near 35 bp upstream of the start point except for 47 bp upstream of the start point (TAGGCA) (Fig. 4) (SEQ ID NO:16). The Shine-Dalgarno ribosomal binding site (AGGAGA) (nucleotides 213-218) was found 9 bp upstream from the initiation codon, ATG (Fig. 4) (SEQ ID NO:16). A terminator-like palindrome sequence consisting of an 11 nucleotide stem with a 4 nucleotide loop structure (stacking energy 24 kcal/mol) was located 9 nucleotides downstream from the stop codon, TGA (Fig. 4) (SEQ ID NO:16). Judging from the secondary structure prediction, this stem-loop structure resembles a p-dependent transcription terminator.

**TABLE I**

| **β-Galactosidase productivity of E-coli transformants** | | |
|---|---|---|
| Strain | β-Galactosidase activity | |
| E.coli JM109 | Activity (U/ml-culture) | Specific activity/(U/mg-protein) |
| /pMC1871 | 0 | 0 |
| /pCHSP | 0.2 | 0.4 |
| 1 U is defined as the amount that produced 1 µmol of α-nitrophenol per h. | | |

### Example 3

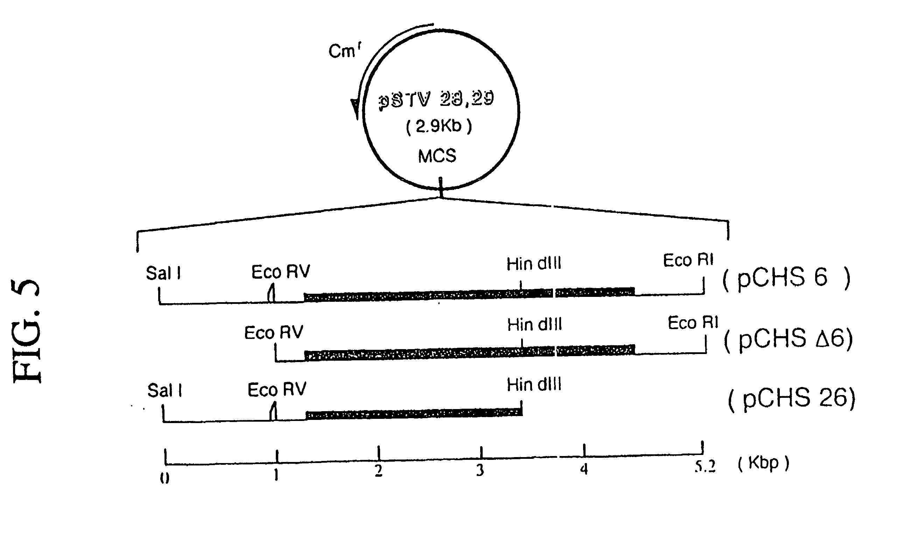

Production of chondroitinase ABC from *E. coli* transformant To demonstrate that the isolated gene codes for chondroitinase ABC, we constructed pCHSΔ6 and pCHS26 (Fig. 5). pCHSΔ6 was constructed by removing the SalI-EcoRV region (about 1kb) upstream from the promoter region from the chondroitinase ABC gene. While pCHS26 was constructed by removing the HindIII-EcoRI region which corresponded to about one third of the 3'-terminal region of the chondroitinase ABC structural gene. These plasmids (pCHS6, pCHSΔ6 and pCHS26) were introduced into *E. coli* XL1-Blue, and *E. coli* transformants were cultured in chondroitin or glucose medium, and chondroitinase ABC *activities* were assayed using the crude extract. The culture fluids of the chondroitin medium were also analyzed to determine degradation products of chondroitin 6-sulfate (Table II). The *E. coli* transformant carrying pCHS6 (containing a 1.0 kb fragment upstream from the promoter) produced the chondroitinase ABC when cultured in chondroitin medium, however, no chondroitinase ABC activity was observed when the transformant was cultured in glucose medium. In contrast, the *E. coli* transformant carrying pCHSΔ6 produced chondroitinase ABC when cultured in either chondroitin or glucose media. The production levels of chondroitinase ABC, cultured in chondroitin media, were 2.6 fold(/pCHS6) and 187 fold(/pCHSΔ6) higher than that of *P. vulgaris*. Even cultured in glucose medium, the production level of chondroitinase ABC in the *E. coli* transformant carrying pCHSΔ6 was 187 fold higher than that of *P. vulgaris* cultured in chondroitin medium. This result suggests that the regulatory sequence might be in the SalI-EcoRV region. Although chondroitin 6-sulfate added to the medium was degraded (p/CHS6 and /pCHSΔ6), *E. coli* transformants were not able to utilize chondroitin sulfate as a carbon source.

**TABLE II**

| **Chondroitinase ABC Activity of E. coli Transformants** | | | | | |
|---|---|---|---|---|---|
| Strain | Intracellular chondroitinase ABC activity | | | | Cultured medium |
| | Chondroitin medium (0.3%) | | Glucose medium (0.3%) | | |
| | Activity ^{a} | Specific ^{b} activity | Activity | Specific activity | Amount of 4,5 Δ chondroitin-6 (µg/ml-culture) |
| E.coli XLI-Blue | 0 | 0 | 0 | 0 | 0 |
| /pSTV29 | 0 | 0 | 0 | 0 | 0 |
| /pCHS6 | 4.1x10⁻³ | 1.6 x10⁻² | 0 | 0 | 192.7 |
| /pCHS26 | 0 | 0 | 0 | 0 | 0 |
| /pCHSΔ6 | 0.3 | 1.2 | 0.3 | 0.5 | 1542.4 |
| *P. vulgeris* | 1.6 x 10⁻³ | 1.2 x 10⁻² | 0 | 0 | 1738.4 |

| | | | | | |
|---|---|---|---|---|---|
| ^{a} I U: enzyme activity producing 1 µmol, 4,5Δ chondroitin-6 per min | | | | | |
| ^{b} U/mg-protein | | | | | |

It has been reported that the *Bacteriodes thetaiotaomicron* chondroitin lyase II gene is adjacent to the chondrosulfatase gene which may be a part of an operon (Guthrie, E. P. et al, (1987) J.Bacteriol. 169: 1192-1199). These same investigators reported that the promoter for this gene recognized in *E coli* may not be the promoter from which the chondroitin lyase II gene is transcribed from in *B. thetaiotaomicron* (Id.) In fact, a putative open reading frame 12 bp upstream from the initiation codon, ATG, was found in the chondroitinase ABC gene (Fig. 4) (SEQ ID NO:16). However, primer extension analysis revealed that the transcription start point is located 41 bp upstream from the initiation codon in *P. vulgaris* (Fig. 3) (SEQ ID NO:15). Even though the chondroitinase ABC gene from *P. vulgaris* cells was also part of an operon, chondroitinase ABC gene was transcribed 41 bp upstream from the initiation codon in *P. vulgaris* cells.

The secondary structure of chondroitinase ABC was estimated by the method of Chou and Fasman (Annu. Rev. Biochem. 47: 251-276 (1978)). A highly complex region was found between amino acid residues 450 and 850. The pCHS26 lacks one-third of the chondroitinase ABC gene encoding the C-terminal region (amino acid residues 646-1021). Removing this region of the enzyme caused the disappearance of chondroitinase ABC activity (Table II). This result suggests that there might be an active site in this region.

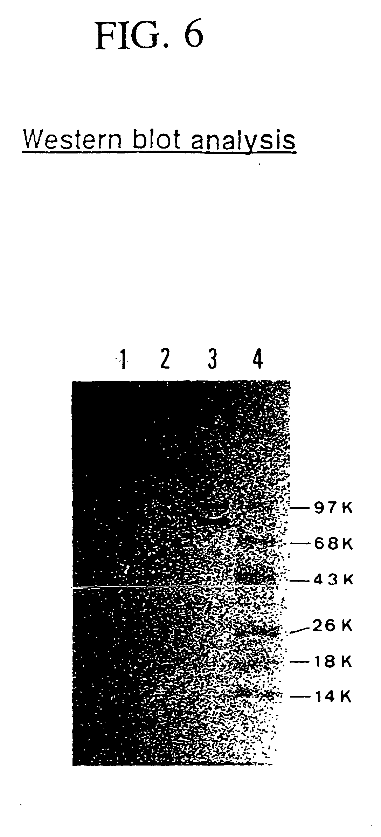

· Recombinant chondroitinase ABC produced by *E. coli* carrying pCHSΔ6 was analyzed by SDS-PAGE followed by immunoblotting (Fig. 6). The immunoblotting patterns of recombinant and native chondroitinase ABC (100 kd) were quite similar. Our previous report showed chondroitinase ABC purified from *P. vulgaris* was a subunit structure consisting of a 90 kd and a 20 kd protein by SDS-PAGE (Sato, N. et al. (1986) Agric. Biol. Chem. 50: 1057-1059), because this subunit protein would not be separated even using gel filtration and other chromatographic techniques. However, by analysis of the N-terminal sequence, we found that the 100 kd protein and the 20 kd protein had the same N-terminal amino acid sequence. By immunoblot analysis, the 80 kd protein also reacts with IgG specific to the 100 kd protein. Furthermore, genomic restriction analysis suggested that chondroitinase ABC gene was a single gene. When we extracted the 100 kd band of chondroitinase ABC from the acrylamide gel and electrophoresed it again in SDS-PAGE, 80 kd and 20 kd bands appeared. The purified chondroitinase ABC contained no protease activity. These results suggest that chondroitinase ABC was partially digested not enzymatically, but physically in the course of sample preparation for SDS-PAGE

### Equivalents

Those skilled in the art will recognize, or be able to ascertain using no more than routine experimentation, numerous equivalents to the specific procedures described herein. Such equivalents are considered to be within the scope of this invention and are covered by the following claims.

## Claims

1. An isolated nucleic acid having a nucleotide sequence coding for a protein with chondroitinase ABC-activity consisting of SEQ ID No:2.

2. An isolated nucleic acid of claim 1 wherein said nucleotide sequence consists of the nucleotide sequence of SEQ ID No:1.

3. An expression vector comprising the nucleotide sequence coding for a protein with chondroitinase ABC-activity consisting of SEQ ID No:2 operably linked to a regulatory sequence.

4. An expression vector of claim 3 wherein said nucleotide sequence consists of the nucleotide sequence of SEQ ID No:1.

5. A host cell transformed with a nucleic acid vector directing the expression of a nucleotide sequence coding for a protein with chondroitinase ABC-activity consisting of SEQ ID No:2.

6. A host cell of claim 5 wherein the cell is eukaryotic.

7. A method of producing a protein with chondroitinase ABC-activity comprising:
culturing a host cell transformed with a nucleic acid vector directing expression of a nucleotide sequence coding for a protein with chondroitinase ABC-activity consisting of SEQ ID No:2 under conditions appropriate for expression; and isolating the protein from the culture.

8. A protein having a chondroitinase ABC-activity consisting of the amino acid sequence of SEQ ID No:2

## Patentansprüche

1. Isolierte Nucleinsäure mit einer Nucleotidsequenz, die für ein Protein mit Chondroitinase ABC-Wirkung codiert, bestehend aus SEQ ID Nr. 2.

2. Isolierte Nucleinsäure nach Anspruch 1, wobei die Nucleotidsequenz aus der Nucleotidsequenz SEQ ID Nr. 1 besteht.

3. Expressionsvektor, umfassend die Nucleotidsequenz, die für ein Protein mit Chondroitinase ABC-Wirkung codiert, bestehend aus SEQ ID Nr. 2, die funktionell mit einer regulatorischen Sequenz verknüpft ist.

4. Expressionsvektor nach Anspruch 3, wobei die Nucleotidsequenz aus der Nucleotidsequenz SEQ ID Nr. 1 besteht.

5. Wirtszelle, die transformiert ist mit einem Nucleinsäure-Vektor, der die Expression einer Nucleotidsequenz steuert, die für ein Protein mit Chondroitinase ABC-Wirkung codiert, bestehend aus SEQ ID Nr. 2.

6. Wirtszelle nach Anspruch 5, wobei die Zelle eukaryontisch ist.

7. Verfahren zur Herstellung eines Proteins mit Chondroitinase ABC-Wirkung, umfassend:
das Kultivieren einer Wirtszelle, die transformiert ist mit einem Nucleinsäure-Vektor, der die Expression einer Nucleotidsequenz steuert, die für ein Protein mit Chondroitinase ABC-Wirkung codiert, bestehend aus SEQ ID Nr. 2, unter Bedingungen, die für die Expression geeignet sind; und Isolieren des Proteins aus der Kultur.

8. Protein mit Chondroitinase ABC-Wirkung, bestehend aus der Aminosäuresequenz SEQ ID Nr. 2.

## Revendications

1. Acide nucléique isolé ayant une séquence de nucléotides codant pour une protéine à activité de chondroïtinase ABC constituée du SEQ ID N° 2.

2. Acide nucléique isolé selon la revendication 1, dans lequel la dite séquence de nucléotides est constituée de la séquence de nucléotides du SEQ ID N°1.

3. Vecteur d'expression comprenant la séquence de nucléotides codant pour une protéine à activité de chondroïtinase ABC constituée du SEQ ID N° 2, liée de façon utilisable à une séquence de régulation.

4. Vecteur d'expression selon la revendication 3, dans lequel la dite séquence de nucléotides est constituée de la séquence de nucléotides du SEQ ID N° 1.

5. Cellule hôte transformée avec un vecteur d'acide nucléique dirigeant l'expression d'une séquence de nucléotides codant pour une protéine à activité de chondroïtinase ABC, constituée du SEQ ID N° 2.

6. Cellule hôte selon la revendication 5, dans laquelle la cellule est eucaryotique.

7. Procédé de production d'une protéine à activité de chondroïtinase ABC comprenant :
la culture d'une cellule hôte transformée avec un vecteur d'acide nucléique dirigeant l'expression d'une séquence de nucléotides codant pour une protéine à activité de chondroïtinase ABC, constituée du SEQ ID N° 2, dans des conditions appropriées pour l'expression, et
l'isolation de la protéine à partir de la culture.

8. Protéine à activité de chondroïtinase ABC constituée de la séquence d'acides aminés du SEQ ID N° 2.
